# EUROPEAN PATENT APPLICATION

(11) **EP 4 834 900 A1**
(43) Date of publication of application: **04.11.2026**
(21) Application number: 24910604.8
(22) Date of filing: 04.12.2024
(51) Int. Cl.: C07D 413/10

(54) **ISOXAZOLINE URACIL COMPOUND CRYSTALLIZATION METHOD AND APPLICATION THEREOF**

(30) Priority: 28.12.2023 CN 202311835052
(71) Applicant: Nantong Jiangshan Agrochemical & Chemicals Co., Ltd., Nantongshi, Jiangsu 226000 (CN); Jiangshan (Yichang) Crop Technology Co., Ltd., Zhijiang City Yichang, Hubei 443200 (CN)
(72) Inventor: DONG, Lei, Nantong, Jiangsu 226000 (CN); WANG, Li, Nantong, Jiangsu 226000 (CN); DU, Hui, Nantong, Jiangsu 226000 (CN); LIN, Yin, Nantong, Jiangsu 226000 (CN); XU, Wei, Nantong, Jiangsu 226000 (CN)
(74) Representative: Cabinet Chaillot
(86) International application number: PCT/CN2024/136894
(87) International publication number: WO 2025/139672

(57) **Abstract**

Disclosed in the present invention is an isoxazoline uracil compound crystallization method, comprising the following steps: preparing an isoxazoline uracil compound, washing an organic layer with water, and performing filtering; evaporating part of an organic solvent A and adding a solvent B; after heating, performing thorough mixing, cooling, and crystallization to obtain a slurry, performing solid-liquid separation, and performing drying to obtain a powdery solid. The present invention optimizes the amount of organic solvent A removed so that the mass ratio of the isoxazoline uracil compound to the organic solvent A in the remaining solution is 5:1-1:5, reducing the content of the solvent in an oily product, increasing the saturation of a target product in the solution, and facilitating further crystallization and purification. Further, the crystal form of the isoxazoline-containing uracil compound can be transformed, so that the isoxazoline-containing uracil compound transforms from an oily substance into a bundle-shaped crystal form or a crystal block, and changes from an oily state to a powdery state, improving transport and material storage during production of the isoxazoline-containing uracil compound and the product usability thereof, and expanding the application range of the product.

## Description

### FIELD OF TECHNOLOGY

The present invention relates to a crystallization method of an isoxazoline uracil compound and C07D, and specifically relates to the preparation field of heterocyclic compounds.

### BACKGROUND

Isoxazoline uracil compounds are a category of herbicides with excellent performance and have efficient herbicidal activity, especially have an excellent control effect on glyphosate-resistant weeds, e.g., *Eleusine indica*. However, isoxazoline uracil compounds in the existing patents are all oily matters; in the process of practical scale-up production, oily matters are readily coagulated at low temperature, resulting in pipe blockage of production equipment and difficulties in discharging materials. These compounds are prone to form amorphous amber-like block solids during storage and thus, are difficultly taken during the formulation of preparation products and metered inconveniently to achieve accurate access. Moreover, amber-like compounds cannot be filled. Even though an amber-like solid is crushed, it still will be agglomerated during storage and transportation to get back to the amber-like state. These properties of the compounds cause various inconveniences during production, filling, storage, transfer, and use.

A Chinese invention patent 201811146442.1 discloses a method for preparing an isoxazoline-containing uracil compound through methylation; the last step of obtaining the product is achieved by removing off solvent under reduced pressure. However, the product prepared by the method is an amorphous sticky oily matter at a slightly high temperature of 25°C above and becomes an amber-like solid after being placed at low temperature. The solid will be also re-agglomerated during storage after being crushed, which causes great inconveniences in production, filling, storage, transfer, and use.

### SUMMARY

To improve the existing state of isoxazoline uracil compounds and solve the defects existing in production, filling, storage, transfer, and use, a first aspect of the present application is to provide a crystallization method of an isoxazoline uracil compound, including the following steps:
(1) preparing the isoxazoline uracil compound, washing an organic layer with water and performing filtering;
(2) distilling off an organic solvent A, and adding a solvent B; and
(3) heating up to a certain temperature, mixing well, cooling and crystallizing to obtain a slurry, and performing solid-liquid separation to obtain a powdery solid after drying.

As a preferred embodiment, the step (2) further includes adding a certain amount of a solvent C for dissolving, and adding the solvent B; alternatively, adding a mixed solution of the solvent B and a solvent C after distilling off the organic solvent A.

As a preferred embodiment, the isoxazoline uracil compound has a structural formula of 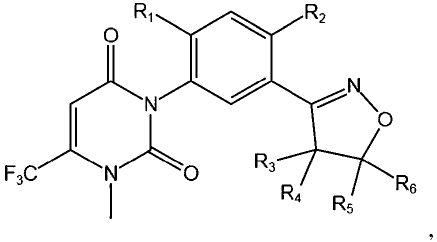 in the formula, R₁ is fluorine, R₂ is chlorine, R₃ and R₄ are hydrogen, R₅ is CO₂C₂H₅, and R₆ is methyl.

As a preferred embodiment, the preparation method of an isoxazoline uracil compound is based on the invention patent 201811146442.1; the organic solvent A is the organic solvent in the patent 201811146442.1; preferably, the organic solvent A is toluene.

As a preferred embodiment, the solvent B has a polarity lower than a polarity of the organic solvent A and the solvent C; preferably, the solvent B is selected from one or a combination of more of an ether, an aliphatic hydrocarbon, carbon tetrachloride, carbon disulfide, and an alkane.

As a preferred embodiment, the solvent B is selected from one or more of petroleum ether, diethyl ether, epoxypropane, n-pentane, cyclopentane, hexane, octane, cyclohexane, n-heptane, isooctane, carbon tetrachloride, and carbon disulfide.

As a preferred embodiment, the solvent B is one of petroleum ether, n-heptane, n-pentane, or cyclopentane.

During the experimental process, it is found by the applicant that when the isoxazoline uracil compound containing an organic solvent is directly distilled and dried, solvent is not completely evaporated, the oily matter cannot be dried, and the product has low purity. One of petroleum ether, n-heptane, n-pentane, or cyclopentane is adopted by the applicant as a solvent B and added to the organic solvent A, which can transform the oily matter into a crystal matter; after solid-liquid separation, it is transformed into powdery solid, thus greatly improving product practicability. It is speculated that the possible reason is as follows: the isoxazoline uracil compound shows an oily state in the organic solvent A due to its particular crystal structure; after being introduced, one of petroleum ether, n-heptane, n-pentane, or cyclopentane having a small polarity is mixed and melted with the organic solvent at a specific temperature such that the isoxazoline uracil compound gets into one of petroleum ether, n-heptane, n-pentane, or cyclopentane. The crystal containing the isoxazoline uracil compound is then arranged orderly and solvent is slowly diffused into the crystal such that the crystal form of the isoxazoline uracil compound is changed and shows an agglomerated bunchy crystal or block crystal, and then it is recrystallized to achieve solid-liquid separation, thus obtaining the powdery solid. It is further found by the applicant that when the solvent B is one of petroleum ether, n-heptane, n-pentane, or cyclopentane, the obtained product has high yield and quantitative content. This is probably because there is a great difference of polarity between the organic solvent A and petroleum ether, n-heptane, n-pentane, or cyclopentane, and a high transformation proportion of the crystal form, which thus can obtain solid powder with high yield and content.

As a preferred embodiment, after the organic solvent A is distilled, a mass ratio of the isoxazoline uracil compound to the organic solvent A in a remaining solution is (1-5):(0-5).

As a preferred embodiment, after the organic solvent A is distilled, a mass ratio of isoxazoline uracil compound to the organic solvent A in a remaining solution is (1-4):(0-4).

As a preferred embodiment, after the organic solvent A is distilled, a mass ratio of isoxazoline uracil compound to the organic solvent A in a remaining solution is 1:1.

As a preferred embodiment, after the organic solvent A is distilled, a mass ratio of isoxazoline uracil compound to the organic solvent A in a remaining solution is 1:0.

It is found by the applicant in the experimental process that the isoxazoline uracil compound is an oily matter; during practical production use, sticky oily matter is readily coagulated at low temperature, resulting in pipe blockage by materials and difficulties in discharging, thus affecting the production of scale-up experiments. It is found by the applicant that after the organic layer containing the oily product is washed with water and filtered, a portion of the organic solvent is distilled off at normal pressure such that a mass ratio of the target product to the organic solvent in the remaining solution is 5:1-1:5, thus increasing the saturability of the target product in solution; hence, the target product can be concentrated into a small volume; when the solvent B is introduced into the solution, the target product carries the organic solvent A to be transferred into the solvent B, thereby forming a system with the solvent B of a continuous phase and the organic solvent A of a dispersion phase, which is beneficial to the mutual solubility and transfer of the subsequent steps. Moreover, it is found by the applicant that when the mass ratio of the target product to the organic solvent is 5:1-1:5, there exists a low difficulty of distillation at normal pressure, and the organic solvent A can be removed by a simple process; if it is beyond the preferred mass ratio, the removal difficulty of the organic solvent A rises perpendicularly. It is speculated that the possible reason is as follows: the oily matter product has good solubility in the organic solvent A, and as the organic solvent A is removed off, the proportion of the oily matter in solution increases gradually and the oily matter will excessively affect the boiling point of the solution, resulting in difficult distillation.

As a preferred embodiment, a mass ratio of an amount of the solvent B added to the organic solvent A in a remaining solution, or to the newly added solvent C is 1:10-10:1.

As a preferred embodiment, a mass ratio of an amount of the solvent B added to the organic solvent A in a remaining solution, or to the newly added solvent C is 1:5-5:1.

As a preferred embodiment, a mass ratio of an amount of the solvent B added to the organic solvent A in a remaining solution, or to the newly added solvent C is 4.3:1.

During the experimental process, it is found by the applicant that the isoxazoline uracil compound can achieve the transformation of its crystal form in a mixed solvent having a mass ratio of 1:10-10:1, and the compound is transformed into a crystal form agglomerated by rodlike crystals from the crystal form of oily matter, thus making it easy to achieve solid-liquid separation and obtaining powdery solid. It is speculated that the possible reason is as follows: in the mixed solvent of the organic solvent A and the solvent B having a mass ratio of 1:10-10:1, isoxazoline uracil compounds are readily arranged orderly to form rodlike crystals to improve the arrangement regularity between the isoxazoline uracil compounds and to reduce the number of the randomly arranged crystals, thereby transforming the oily matter into an aggregate of rodlike crystals. The number of the orderly arranged crystals is up to the maximum within the scope of the preferred weight ratio such that the obtained solid powder has a higher yield after solid-liquid separation. Moreover, a high transformation rate of crystal form also achieves a significantly increased content of the target product, thus increasing the quantitative content of the target product.

As a preferred embodiment, a temperature rise in the step (3) is not greater than a boiling point of a mixed solution of the organic solvent A and the solvent B, and preferably, the temperature rise is 30-95°C.

As a preferred embodiment, a temperature rise in the step (3) is not greater than a boiling point of a mixed solution of the organic solvent A and the solvent B, and preferably, the temperature rise is 35-60°C.

As a preferred embodiment, a temperature rise in the step (3) is not greater than a boiling point of a mixed solution of the organic solvent A and the solvent B, and preferably, the temperature rise is 40°C.

During the experimental process, it is found by the applicant that the isoxazoline uracil compound has a low solubility in the solvent B; the rise of temperature can increase the solubility of the isoxazoline uracil compound in the solvent B; when temperature reaches 40-70°C, the solution can be up to a homogeneous phase state to achieve complete mutual dissolution; and the isoxazoline uracil compound containing the organic solvent can be completely dissolved and dispersed into the solvent B, thus achieving the transformation of crystal form. It is speculated that the possible reason is as follows: the oily isoxazoline uracil compound has poor dispersibility in the solvent B with low polarity; by rising temperature, intermolecular activity increases and intermolecular distance enlarges for transformation of crystal form. The solvent B is permeated into gaps of the crystal containing the isoxazoline uracil compound to improve the solubility of the isoxazoline uracil compound into the mixed solution of one of petroleum ether, n-heptane, n-pentane, or cyclopentane with the solvent A, thus increasing the transformation rate of crystal form. It is further found by the applicant that when the temperature rise exceeds the preferred temperature, boiling readily occurs between the solvents to increase the volatile quantity of one of petroleum ether, n-heptane, n-pentane, or cyclopentane; the transformation amount of the crystal form containing the isoxazoline uracil compound declines with the increase of the petroleum ether, n-heptane, n-pentane, or cyclopentane in volatile quantity, leading to declined quantitative content of the final product and reducing the final yield of the target product.

As a preferred embodiment, in the step (3), the cooling and crystallizing are performed by: cooling to 0-10°C within 2-5 hours, and performing heat-preservation crystallization for 1-6 hours.

As a preferred embodiment, in the step (3), the cooling and crystallizing are performed by: cooling to 5°C within 2-4 hours, and performing heat-preservation crystallization for 4-5 hours.

As a preferred embodiment, in the step (3), the cooling and crystallizing are performed by: cooling to 5°C within 3 hours, and performing heat-preservation crystallization for 5 hours.

As a preferred embodiment, the solvent C is selected from one or a combination of more of an aromatic hydrocarbon organic solvent, an alicyclic hydrocarbon organic solvent, an ester organic solvent, a ketone organic solvent, and a halogenated hydrocarbon organic solvent.

As a preferred embodiment, the solvent C is selected from one or a combination of more of benzene, toluene, xylene, chlorobenzene, dichlorobenzene, ethyl acetate, ethyl oleate, propyl acetate, isopropyl acetate, methyl acetate, acetone, methylbutanone, methyl isobutyl ketone, methyl ethyl ketone, dichloromethane, 1,2-dichloroethane, chloroform, bromoethane, chloropropane, and methyl oleate.

As a preferred embodiment, the solvent C is ethyl acetate.

A second aspect of the present invention is to provide use of the crystallization method of the isoxazoline uracil compound, for use in the crystallization preparation of the isoxazoline uracil compound.

Compared with the prior art, the present invention has the following advantageous effects:
(1) According to the crystallization method of the isoxazoline uracil compound in the present invention, a removal amount of the organic solvent A is preferably configured such that a mass ratio of the isoxazoline uracil compound to the organic solvent A in a remaining solution is 5:1-1:5, which reduces the content of the solvent in an oily product, increases the saturability of the target product in solution, and is beneficial to further crystallization and purification.
(2) According to the crystallization method of the isoxazoline uracil compound in the present invention, one of petroleum ether, n-heptane, n-pentane, or cyclopentane having a polarity lower than that of the organic solvent A is adopted as the solvent B, which can transform the crystal form containing the isoxazoline uracil compound into a bunchy crystal form or crystal block from an oily matter, thus being further transformed into powdery solid after solid-liquid separation.
(3) According to the crystallization method of the isoxazoline uracil compound in the present invention, adopting a temperature rise not exceeding the boiling point of the mixed solvent can increase the solubility of the isoxazoline uracil compound in one of petroleum ether, n-heptane, n-pentane, or cyclopentane, increase the transformation rate of crystal form, reduce the volatilization of solvent to avoid affecting the recrystallization due to the reduction of solvent.
(4) According to the crystallization method of the isoxazoline uracil compound in the present invention, the mass ratio of the amount of the added solvent B to the organic solvent A in the remaining solution is 1:10-10:1, which can increase the yield of the final product powder solid and increase the quantitative content of the target product in the solid powder.
(5) According to the crystallization method of the isoxazoline uracil compound in the present invention, the isoxazoline uracil compound is transformed into a powdery solid from an oily matter, thus improving the storage and applicability of the isoxazoline uracil compound, and expanding the application range of the product.

### DESCRIPTION OF THE EMBODIMENTS

The present invention will be described specifically through the examples below. It is necessary to indicate that the following examples are merely for the further specification of the prevent invention, but are not construed as limiting the scope of protection of the present invention. Some non-essential improvements and adjustments made by those skilled in the art according to the above disclosure of the present invention shall still fall within the scope of protection of the present invention.

In addition, other raw materials are commercially available other than the isoxazoline uracil compound, unless otherwise specified.

### Example 1

A crystallization method of an isoxazoline uracil compound includes the following steps:
(1) preparing the isoxazoline uracil compound, washing an organic layer with water, and performing filtering;
(2) distilling off a portion of an organic solvent A, and adding a solvent B; and
(3) heating up to a certain temperature, mixing well, cooling and crystallizing to obtain a slurry, and performing solid-liquid separation to obtain a powdery solid after drying.

The preparation method of the isoxazoline uracil compound is based on the invention patent 201811146442.1. 464 g of 3-(2-chloro-5-(2,6-dioxo-4-trifluoromethyl-3,6-dihydropyrimidin-1(2H)-yl)-4-fluorophenyl)-5-methyl-4,5-dihydroisoxaz ole-5-ethyl carboxylate were reacted with 46.6 g of methyl chloride in 2,000 g of toluene; at the end of the reaction, a toluene layer was obtained after washing with water and was divided into four parts averagely.
S1, one part was taken and distilled at normal pressure to remove 384 g of the organic solvent toluene; 498 g of solvent B petroleum ether were dropwise added to the toluene solution of the product under stirring; afterwards, the resulting solution was heated up to 60°C and thermally preserved for 30 min, and cooled to 0°C within 3 h, thermally preserved and crystallized for 5 h, and subjected to solid-liquid separation, and then solid was dried to obtain the product;
S2, the second part was taken and distilled at normal pressure to remove 384 g of the organic solvent toluene; 498 g of solvent B cyclopentane were dropwise added to the toluene solution of the product under stirring; afterwards, the resulting solution was heated up to 60°C and thermally preserved for 30 min, and cooled to 0°C within 3 h, thermally preserved and crystallized for 5 h, and subjected to solid-liquid separation, and then solid was dried to obtain the product;
S3, the third part was taken and distilled at normal pressure to remove 384 g of the organic solvent toluene; 498 g of solvent B n-pentane were dropwise added to the toluene solution of the product under stirring; afterwards, the resulting solution was heated up to 60°C and thermally preserved for 30 min, and cooled to 0°C within 3 h, thermally preserved and crystallized for 5 h, and subjected to solid-liquid separation, and then solid was dried to obtain the product; and
S4, the fourth part was taken and distilled at normal pressure to remove 384 g of the organic solvent toluene; 498 g of solvent B n-heptane were dropwise added to the toluene solution of the product under stirring; afterwards, the resulting solution was heated up to 60°C and thermally preserved for 30 min, and cooled to 0°C within 3 h, thermally preserved and crystallized for 5 h, and subjected to solid-liquid separation, and then solid was dried to obtain the product.

### Example 2

Specific steps of the crystallization method of the isoxazoline uracil compound are the same as those in Example 1. Example 2 differs from Example 1 in that the organic solvent A is ethyl acetate.

### Example 3

A crystallization method of an isoxazoline uracil compound includes the following steps:
(1) preparing the isoxazoline uracil compound, washing an organic layer with water, and performing filtering;
(2) adding a certain amount of a solvent C for dissolving, and adding a solvent B; and
(3) heating up to a certain temperature, mixing well, cooling and crystallizing to obtain a slurry, and performing solid-liquid separation to obtain a powdery solid after drying.

The preparation method of the isoxazoline uracil compound is based on the invention patent 201811146442.1. 464 g of 3-(2-chloro-5-(2,6-dioxo-4-trifluoromethyl-3,6-dihydropyrimidin-1(2H)-yl)-4-fluorophenyl)-5-methyl-4,5-dihydroisoxazole-5-ethyl carboxylate were reacted with 46.6 g methyl chloride; all the organic solvent A toluene were distilled off to prepare 484 g of oily matter having a quantitative content of 92.8%, and the oily matter was divided into four parts averagely.
S1, one part of the oily matter was taken and dissolved with 133.1 g of the solvent C ethyl acetate to form a homogeneous phase solution; 266.2 g of solvent B petroleum ether were dropwise added, the resulting solution was heated up to 50°C and thermally preserved for 30 min to form a homogeneous phase solution, then cooled to 0°C within 3 h, thermally preserved and crystallized for 5 h, and subjected to solid-liquid separation, and then solid was dried to obtain the product;
S2, the second part of the oily matter was taken and dissolved with 133.1 g of the solvent C ethyl acetate to form a homogeneous phase solution; 266.2 g of solvent B cyclopentane were dropwise added, the resulting solution was heated up to 50°C and thermally preserved for 30 min to form a homogeneous phase solution, then cooled to 0°C within 3 h, thermally preserved and crystallized for 5 h, and subjected to solid-liquid separation, and then solid was dried to obtain the product;
S3, the third part of the oily matter was taken and dissolved with 133.1 g of the solvent C ethyl acetate to form a homogeneous phase solution; 266.2 g of solvent B n-pentane were dropwise added, the resulting solution was heated up to 50°C and thermally preserved for 30 min to form a homogeneous phase solution, then cooled to 0°C within 3 h, thermally preserved and crystallized for 5 h, and subjected to solid-liquid separation, and then solid was dried to obtain the product; and
S4, the fourth part of the oily matter was taken and dissolved with 133.1 g of the solvent C ethyl acetate to form a homogeneous phase solution; 266.2 g of solvent B n-heptane were dropwise added, the resulting solution was heated up to 50°C and thermally preserved for 30 min to form a homogeneous phase solution, then cooled to 0°C within 3 h, thermally preserved and crystallized for 5 h, and subjected to solid-liquid separation, and then solid was dried to obtain the product.

### Performance test

Solid prepared in Examples 1-3 were weighed, and the quantitative content of the target products was determined by HPLC. Examples 1-3 are corresponding to Tables 1-3, respectively.

**Table 1**

| | S1 | S2 | S3 | S4 |
|---|---|---|---|---|
| Mass/g | 109.95 | 111.20 | 110.80 | 112.30 |
| Quantitative content/% | 98.12 | 97.95 | 98.02 | 98.02 |

**Table 2**

| | S1 | S2 | S3 | S4 |
|---|---|---|---|---|
| Mass/g | 110.32 | 110.35 | 111.20 | 111.65 |
| Quantitative content/% | 98.10 | 98.08 | 97.90 | 97.96 |

**Table 3**

| | S1 | S2 | S3 | S4 |
|---|---|---|---|---|
| Mass/g | 107.15 | 107.46 | 107.25 | 108.20 |
| Quantitative content/% | 98.51 | 98.23 | 98.41 | 97.56 |

## Claims

1. A crystallization method of an isoxazoline uracil compound, comprising the following steps:
(1) preparing the isoxazoline uracil compound, washing an organic layer with water, and performing filtering;
(2) distilling off an organic solvent A, and adding a solvent B; and
(3) heating up to a certain temperature, mixing well, cooling and crystallizing to obtain a slurry, and performing solid-liquid separation to obtain a powdery solid after drying.

2. The crystallization method of an isoxazoline uracil compound according to claim 1, wherein the step (2) further comprises adding a certain amount of a solvent C for dissolving, and adding the solvent B; alternatively, adding a mixed solution of the solvent B and a solvent C after distilling off the organic solvent A.

3. The crystallization method of an isoxazoline uracil compound according to claim 1, wherein the isoxazoline uracil compound has a structural formula of 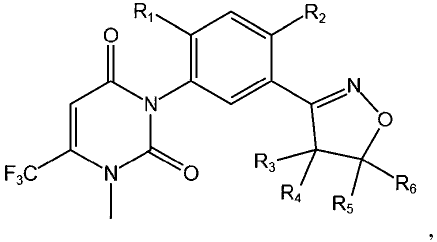 wherein in the formula, R₁ is fluorine, R₂ is chlorine, R₃ and R₄ are hydrogen, R₅ is CO₂C₂H₅, and R₆ is methyl.

4. The crystallization method of an isoxazoline uracil compound according to claim 2, wherein the solvent B has a polarity lower than a polarity of the organic solvent A and the solvent C; preferably, the solvent B is selected from one or a combination of more of an ether, an aliphatic hydrocarbon, carbon tetrachloride, and carbon disulfide.

5. The crystallization method of an isoxazoline uracil compound according to claim 2, wherein after the organic solvent A is distilled, a mass ratio of the isoxazoline uracil compound to the organic solvent A in a remaining solution is (1-5):(0-5).

6. The crystallization method of an isoxazoline uracil compound according to claim 2, wherein a mass ratio of an amount of the solvent B added to the organic solvent A in a remaining solution, or to the newly added solvent C is 1:10-10:1.

7. The crystallization method of an isoxazoline uracil compound according to claim 2, wherein a temperature rise in the step (3) is not greater than a boiling point of a mixed solution of the organic solvent A and the solvent B, and preferably, the temperature rise is 30-95°C.

8. The crystallization method of an isoxazoline uracil compound according to claim 1, wherein in the step (3), the cooling and crystallizing are performed by: cooling to 0-10°C within 2-5 hours, and performing heat-preservation crystallization for 1-6 hours.

9. The crystallization method of an isoxazoline uracil compound according to claim 2, wherein the solvent C is selected from one or a combination of more of an aromatic hydrocarbon organic solvent, an alicyclic hydrocarbon organic solvent, an ester organic solvent, a ketone organic solvent, and a halogenated hydrocarbon organic solvent.

10. Use of the crystallization method of an isoxazoline uracil compound according to any one of claims 2-9, for use in crystallization preparation of the isoxazoline uracil compound.
